(19) Europäisches Patentamt
European Patent Office
Office européen des brevets

(11) Numéro de publication: **0 394 112 A2**

(12) **DEMANDE DE BREVET EUROPEEN**

(21) Numéro de dépôt: **90401021.2**

(51) Int. Cl.5: **A61K 31/475**

(22) Date de dépôt: **13.04.90**

(30) Priorité: **17.04.89 FR 8905068**

(43) Date de publication de la demande:
**24.10.90 Bulletin 90/43**

(84) Etats contractants désignés:
**AT BE CH DE DK ES FR GB IT LI LU NL SE**

(71) Demandeur: **INSTITUT NATIONAL DE LA SANTE ET DE LA RECHERCHE MEDICALE (INSERM)**
**101, rue de Tolbiac**
**F-75654 Paris Cédex 13(FR)**

(72) Inventeur: **Chermann, Jean-Claude**
**8 Les Ombrées 1, Route de la Treille**
**F-13011 Marseille(FR)**
Inventeur: **Marchetto, Sylvie**
**Clos Gyptis, 18 avenue Taine**
**F-13009 Marseille(FR)**

(74) Mandataire: **Gillard, Marie-Louise et al**
**Cabinet Beau de Loménie 55, Rue d'Amsterdam**
**F-75008 Paris(FR)**

(54) **Utilisation de dipyrido (4,3-b)(3,4-f) indoles pour la préparation de médicaments utiles pour le traitement du SIDA.**

(57) La présente invention concerne l'utilisation de dipyrido [4,3-b] [3,4-f] indoles de formule :

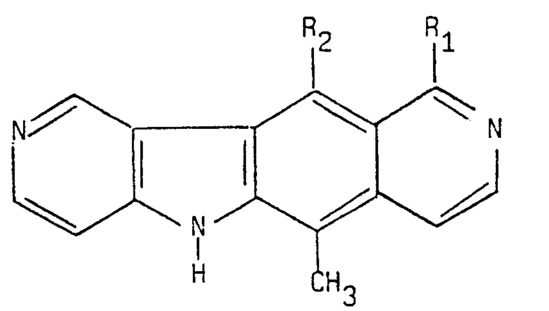

I

dans laquelle : - $R_1$ est l'hydrogène, le groupe hydroxy, un groupe alkyle en $C_1$-$C_6$, un groupe alkyl($C_1$-$C_6$)-thio, un groupe alcoxy en $C_1$-$C_6$, un halogène, de préférence le chlore ou un groupe amino non substitué ou substitué par un groupe de formule :

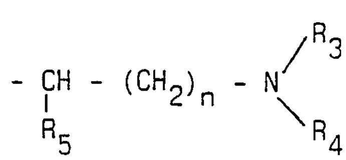

dans laquelle $R_3$ et $R_4$ identiques ou différents, représentent chacun l'hydrogène ou un radical alkyle en $C_1$-$C_6$, $R_5$ représente l'hydrogène ou un radical alkyle en $C_1$-$C_6$ et n représente un nombre entier de 1 à 10 ;-

EP 0 394 112 A2

$R_2$ est l'hydrogène ou un groupe alkyle en $C_1$-$C_4$, et de leurs sels pharmaceutiquement acceptables pour la préparation de médicaments utiles pour le traitement du SIDA.

**Utilisation de dipyrido [4,3-b] [3,4-f] indoles pour la préparation de médicaments utiles pour le traitement du SIDA.**

La présente invention a pour objet l'utilisation de dipyrido [4,3-b] [3,4-f] indoles pour la préparation de médicaments utiles pour le traitement de l'immunodéficience humaine, également connue sous le nom de SIDA.

Le SIDA ou syndrome d'immunodéficience acquise est la phase terminale d'une maladie, connaissant actuellement une expansion épidémique, dont l'agent responsable est l'un des rétrovirus de l'immunodéficience humaine ou virus HIV (HIV 1 ou HIV 2).

Les virus HIV, transmis notamment par voie sexuelle ou à l'occasion d'une transfusion sanguine avec du sang contaminé, ont pour cibles les cellules ayant à leur surface un récepteur antigénique connu sous la dénomination de molécule de surface CD4 ou récepteur CD4. Les lymphocytes T4 qui jouent un rôle essentiel dans le système immunitaire, possèdent ce récepteur CD4 et sont donc des cellules-cibles des virus HIV.

L'infection par les virus HIV débute donc par la fixation des particules virales, et plus particulièrement de la protéine gp 120 de l'enveloppe virale, sur les récepteurs CD4 des lymphocytes T4. Elle se poursuit par la fusion de l'enveloppe virale et de la membrane cellulaire ; l'ARN du génome viral est ensuite transcrit à l'aide d'une enzyme particulière, la transcriptase inverse ou reverse transcriptase (qui n'existe pas dans les cellules normales) en ADN double brin et intégré au génome cellulaire.

Une cellule ainsi infectée synthétise alors les protéines virales et notamment la protéine gp120, qui se loge sur la membrane cellulaire. Du fait de la grande affinité de la protéine gp 120 pour les récepteurs CD4, cette cellule infectée peut fixer des lymphocytes T4 sains et former un syncytium incapable de survivre. Une seule cellule infectée peut donc provoquer la mort de nombreux lymphocytes T4 sains.

D'autre part, des protéines virales gp 120 isolées circulent parfois dans le sang et la lymphe et se lient aux récepteurs CD4 des lymphocytes T4 sains, lesquels deviennent alors des cellules-cibles pour le système immunitaire.

Les virus HIV se répliquent aussi dans les monocytes, les macrophages et les cellules apparentées et perturbent probablement leur fonctionnement. L'infection par un virus HIV peut d'une certaine manière modifier la quantité ou la structure des cytokines produites par les macrophages activés et les lymphocytes activés, ces cytokines s'avérant alors toxiques pour les lymphocytes T4 auxiliaires.

L'un quelconque des mécanismes ci-dessus entraîne la destruction des lymphocytes T4 et simultanément l'effondrement du système immunitaire.

Différents moyens d'action sur les virus HIV ont déjà été envisagés, notamment au niveau de la phase de fixation des virus sur les cellules-cibles ou au niveau de la replication des virus.

A cet effet, on peut se référer notamment à l'ouvrage de F. BARRE-SINOUSSI et al. intitulé "LE SIDA EN QUESTION", Collection Témoins d'Aujourd'hui, édité par PLON (1987) et à l'article de R. YARCHOAN et al. intitulé "LES TRAITEMENTS DU SIDA", dans la Revue "Pour la Science" Décembre 1988, p. 94-104.

Les inhibiteurs de la transcriptase inverse actuellement utilisés dans le traitement du SIDA sont des didésoxynucléosides, qui sont des analogues des nucléosides, et notamment l'azidothymidine (AZT).

Le problème avec ces inhibiteurs est qu'ils ne sont jamais totalement spécifiques et qu'à certaines doses ils peuvent empêcher la division des cellules non infectées, entraînant ainsi des toxicités importantes, nécessitant l'interruption du traitement.

On a également proposé l'utilisation d'oligonucléosides, complémentaires d'une partie des ARN messagers viraux.

On a maintenant trouvé que l'on peut utiliser dans le traitement du SIDA, des dipyrido [4,3-b] [3,4-f] indoles.

Ces dipyrido [4,3-bJ [3,4-f] indoles répondent à la formule :

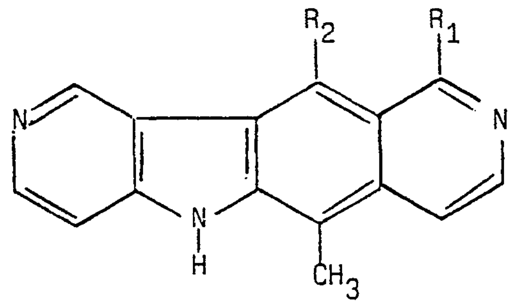

I

dans laquelle :

- $R_1$ est l'hydrogène, le groupe hydroxy, un groupe alkyle en $C_1$-$C_6$, un groupe alkyl($C_1$-$C_6$)thio, un groupe alcoxy en $C_1$-$C_6$, un halogène, de préférence le chlore ou un groupe amino non substitué ou substitué par un groupe de formule :

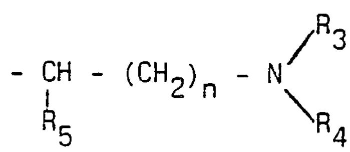

dans laquelle $R_3$ et $R_4$ identiques ou différents, représentent chacun l'hydrogène ou un radical alkyle en $C_1$-$C_6$, $R_5$ représente l'hydrogène ou un radical alkyle en $C_1$-$C_6$ et n représente un nombre entier de 1 à 10 ;

- $R_2$ est l'hydrogène ou un groupe alkyle en $C_1$-$C_4$.

Ces composés, qui présentent des propriétés cytotoxiques et antitumorales remarquables, ont été décrits pour la première fois dans le brevet FR 2.387.229 et fait l'objet de nombreuses publications.

L'activité antitumorale de ces composés sur la leucémie L 1210 a été décrite notamment par :

- J.C. CHERMANN et al. dans C.R. Acad. Sc. Paris, 1977, 285, série D, 945-948 ;
- R. LIDEREAU et al. dans Bull. Cancer (Paris) 1980, 67,1, 1-8 ;
- J.C. CHERMANN et al. dans Proc. Xth Intern. Congr. Chemother., 1978, Vol. 1, 1200-1201.

Les propriétés antitumorales de ces composés ont également été observées dans le cas de la leucémie de Friend, du mélanome B16, du carcinome de Lewis et des tumeurs induites par la souche Moloney du virus du sarcome murin.

Un composé particulièrement intéressant de cette série est le composé de formule I dans lequel $R_1$ est le groupe de formule $-NH-(CH_2)_3-N(C_2H_5)_2$ et $R_2$ est l'hydrogène. Ce composé, le 1-[(γ-diéthylaminopropyl)amino]-5-méthyl-dipyrido [4,3-bJ [3,4-f] indole, est bien connu sous la dénomination BD 40.

Ce composé est également dénommé [[(diéthylamino)-3 propyl] amino] -10 méthyl-6 5H-pyrido [3′, 4′ : 4,5] pyrrolo [2,3-g] isoquinoléine et possède maintenant la dénomination commune internationale "PAZELLIPTINE".

Les effets du BD 40 ont été également étudiés sur des cellules en culture (voir M. J. VILAREM et al., Biochimie, 1982, 64, 923-932). Il a été constaté que la viabilité et la morphologie des cellules (lignée cellulaire murine D55 et fibroblastes murins NIH - 3T3) sont affectées de manière irréversible par des traitements pendant 24 heures à l'aide du BD 40 à des concentrations égales ou supérieures à 0,10 µM.

On a attribué de telles propriétés au pouvoir du BD 40 de s'intercaler dans l'ADN (voir notamment E. MOUSTACCHI et al. Cancer research, 1983, 43, 3700-3706 et TOURBEZ-PERRIN et al. Bull. Cancer (Paris), 1980, 67,1 , 9-13).

On a maintenant trouvé, de façon surprenante, que le BD 40 et les composés apparentés inhibent notamment la transcriptase inverse des virus HIV, agent responsable du SIDA ainsi que la production virale des lymphocytes périphériques infectés par les virus HIV.

On a également constaté que le BD 40 est capable d'inhiber la réplication du virus HIV au sein même des macrophages, effet qui devrait contribuer à limiter la propagation du virus HIV à laquelle participent ces cellules.

Ainsi, la présente invention a pour objet l'utilisation des dipyrido [4,3-b] [3,4-f] indoles de formule I, telle que définie précédemment ou de leurs sels pharmaceutiquement acceptables pour la préparation de médicaments utiles pour le traitement du SIDA.

Les composés de formule I ci-dessus, dans laquelle :

- $R_1$ est l'hydrogène, le chlore ou un groupe de formule

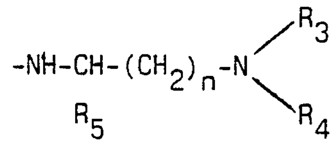

dans laquelle n est un nombre entier compris entre 1 et 4, $R_3$ et $R_4$ identiques ou différents, représentent l'hydrogène ou un groupe alkyle en $C_1$-$C_6$, de préférence en $C_1$-$C_4$ ; $R_5$ est l'hydrogène ou un groupe alkyle en $C_1$-$C_4$, de préférence le groupe méthyle,

- $R_2$ est l'hydrogène ou un groupe alkyle en $C_1$-$C_4$, de préférence le groupe méthyle, ainsi que leurs sels pharmaceutiquement acceptables, sont des composés préférés aux fins de l'invention ; le BD 40, notamment sous forme de trimaléate ou de trichlorhydrate, étant particulièrement préféré.

Ces composés peuvent être obtenus notamment par les procédés décrits dans le brevet FR 2.387.229.

Les doses à utiliser dépendent du stade de l'évolution de la maladie.

On utilisera avantageusement les dipyrido [4,3-b] [3,4-f] indoles par voie intraveineuse. Par exemple dans le cas de patients souffrant d'un SIDA avéré qui s'exprime sous la forme d'un sarcome de Kaposi, des injections de trichlorhydrate de BD 40 en perfusion de 50 à 300 mg/m²/jour par cures de 3 jours administrées à 3 semaines d'intervalle l'une après l'autre, sont appropriées.

L'invention va maintenant être décrite plus en détail par les tests ci-après.

## I/ - Dosage de la transcriptase inverse

Le trichlorhydrate de BD 40 (sel ci-après appelé "BD 40"), obtenu par réaction de la base BD 40 en phase aqueuse avec une solution aqueuse d'acide chlorhydrique, a été testé dans un premier temps en tant qu'inhibiteur direct sur l'activité enzymatique de la transcriptase inverse du virus HIV 1.

L'activité enzymatique a été testée en utilisant une amorce synthétique et un substrat radiomarqué au $H^3$ "in vitro" en présence de différentes doses du BD 40. La quantité de matériel radiomarqué précipité, exprimée en coups par minute, est proportionnelle à l'activité de la transcriptase inverse.

Les résultats obtenus figurent sur le graphe de la figure 1, sur laquelle on a porté en ordonnées, la quantité de matériel radiomarqué en coups par minute (RT en cpm) et en abscisses les quantités de BD 40 testées.

On constate que l'activité de la transcriptase inverse du virus HIV est totalement inhibée par le BD 40 aux concentrations les plus élevées, de 1 mg/ml à 50 $\mu$g/ml.

A partir de la concentration en BD 40 de 5 $\mu$g/ml on observe une activité de l'enzyme du même niveau qu'en l'absence d'inhibiteur.

## II/ - Test d'infectivité de lymphocytes périphériques

Les lymphocytes obtenus après stimulation à la PHA (Phytohémagglutinine) de cellules issues d'une cytaphérèse, ont été infectés par une dilution appropriée de virus HIV 1 afin d'obtenir un pic de production virale aux environs du quinzième jour après infection. Différentes doses du BD 40 ont été ajoutées au milieu de culture au moment de l'infection et pendant toute la culture afin de déterminer les doses toxiques et protectrices.

Pour déterminer le mécanisme d'action du composé, celui-ci a été ajouté à la culture soit pendant une période de préincubation des cellules avant infection, soit pendant toute la période de culture suivant l'infection.

Le suivi de la production virale des lymphocytes a été réalisé par dosage de l'activité de la transcriptase inverse dans la culture tous les 3 ou 4 jours.

Les résultats obtenus, avec le BD 40 à la concentration de 12,5 ng/ml, figurent sur les différentes courbes de la figure 2 qui expriment l'activité de la transcriptase inverse (RT en ordonnées) en fonction du temps en jours.

Fig. 2a : BD 40 ajouté pendant la période de préincubation des cellules avant infection.

Fig. 2b : BD 40 ajouté pendant la culture qui suit l'infection.
Fig. 2c : BD 40 ajouté à la culture, uniquement après l'infection.
Fig. 2d : Pas de BD 40. Contrôle HIV 1.

En examinant ces courbes, on observe que la présence du BD 40 à la concentration de 12,5 ng/ml, lors d'une préincubation d'une heure avant l'infection (Fig. 2a), suffit à inhiber totalement la production virale de lymphocytes infectés. Le même résultat est obtenu lorsque le BD 40 est présent aussi pendant la culture qui suit l'infection (Fig. 2b). L'addition du BD 40 à la culture (Fig. 2c) uniquement après l'infection, fait apparaître un pic de production virale qui reste d'un niveau plus faible que le pic obtenu en l'absence d'inhibiteur (Fig. 2d).

## III/ - Action du BD 40 sur la production du virus HIV par les lymphocytes périphériques

Ce test a été réalisé comme suit.

Les lymphocytes humains ont été isolés sur gradient de Ficoll à partir du sang périphérique d'un donneur séronégatif. Préalablement au traitement, ils ont été stimulés par la phytohémagglutinine p (PHA-p) (Difco) pendant 3 jours. Ils sont ensuite maintenus en culture dans un milieu RPMI sans glutamine (GIBCO, Réf. 041.01870M, contenant 10 % de sérum de veau fétal (FLOW, Réf. 29.101.711), 1 % de glutamine (GIBCO, Réf. 043.05030H), 1 % d'une solution d'antibiotiques (GIBCO, PSN, Réf. 043.05640H), 10 % d'une solution d'interleukine 2 (BIOTEST, Réf. 81.1020), et 2g/ml de polybrène (SIGMA, Réf. 36F.3686 P. 4515).

Du trichlorhydrate de BD 40 (sel ci-après appelé BD 40) est ajouté aux doses non toxiques de 3,2 jusqu'à 25 ng/ml en même temps que l'infection virale. La production du virus dans le surnageant est mesurée par l'activité réverse transcriptase après concentration des cellules infectées par centrifugation.

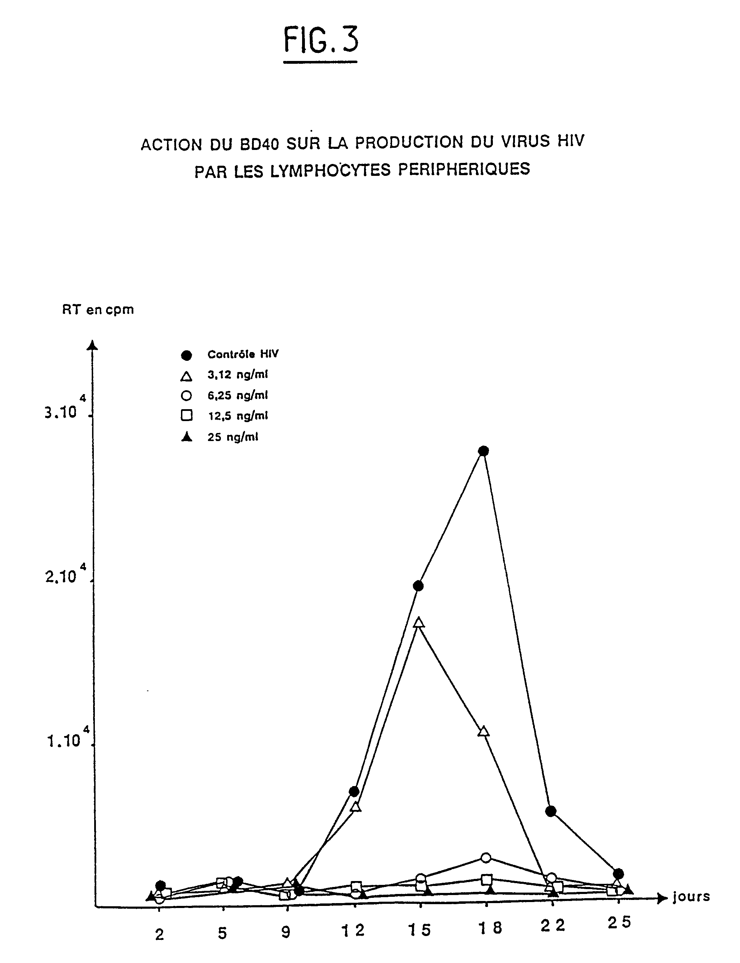

Les résultats obtenus figurent sur les courbes de la figure 3 qui donne l'activité de transcriptase inverse (RT en ordonnées) en fonction du temps pour des quantités données de BD 40.

Pour des concentrations non toxiques en composé BD 40 de 25 ng/ml à 6,25 ng/ml, on observe une inhibition très importante de la production virale des lymphocytes périphériques infectés. A la concentration de 3,12 ng/ml, un pic de production virale apparait d'un niveau inférieur à celui obtenu en l'absence d'inhibiteur.

Les résultats obtenus dans les différents types d'expériences montrent que le composé BD 40 est un puissant inhibiteur de la transcriptase inverse du virus HIV 1.

De plus, ce composé est capable, à des concentrations très faibles, d'inhiber la production virale de lymphocytes périphériques infectés par le virus HIV 1.

L'absence totale de virus produit jusqu'au jour 42 pour les doses les plus élevées et non toxiques, ainsi que les expériences de préincubation des cellules avec le BD 40 avant infection, montrent que le composé BD 40 agit à des stades très précoces de l'infection par le virus HIV 1.

On peut penser que par son action inhibitrice sur l'activité de la transcriptase inverse, il empêche la transcription de l'ARN viral et donc son intégration au génôme cellulaire.

## IV/ - Action inhibitrice du BD 40 sur la réplication du virus HIV 1 dans les macrophages

Les macrophages ont été isolés à partir du sang périphérique par adhérence. On les a infectés par du virus HIV 1 (de 5000 à 25000 cpm (RT en cpm par ml)). 3 à 7 jours après, les macrophages ont été éclatés par congélation/décongélation et le virus a été révélé par une coculture avec une lignée lymphoblastoïde (CEM ou lymphocytes du sang périphérique).

Différentes doses de trichlorhydrate de BD 40 (ci-après BD 40) ont été testées, le produit étant ajouté au milieu de culture au moment de l'infection.

Dans ces conditions expérimentales, alors que l'infection virale témoin montre une production de virus dans les 3 à 7 jours après la coculture, par contre pour les macrophages traités avec le BD 40 on constate une diminution du nombre de virus, par mesure de l'activité réverse-transcriptase, et un retard d'apparition confirmant ainsi une inhibition partielle de la réplication du virus. Cette inhibition se traduit par un retard de 2 jours aux doses non toxiques de 10 et 15 ng/ml de BD 40.

Ces résultats mettent donc bien en évidence que l'action du BD 40 sur l'inhibition de la réplication du virus HIV ne se limite pas aux seuls lymphocytes mais concerne également les macrophages infectés.

## V/ - Inhibition de la réserve transcriptase du virus HIV chez les malades traités par le BD 40

**trichlorhydrate**

Lors d'un essai clinique chez 12 malades souffrant d'un SIDA avéré avec sarcome de Kaposi traités par des injections de trichlorhydrate de BD 40 en perfusion à des doses comprises entre 50 et 300 mg/m$^2$/jour par cures de 3 jours consécutifs à intervalles de 3 semaines, des mesures de l'activité réverse transcriptase (RT) dans les cellules sanguines ont été pratiquées avant administration et aux 4e et 15e jours après le début de chaque cure.

La technique de mesure est la même que celle utilisée dans les tests II et III ci-dessus. Les résultats obtenus sont les suivants :

- 8 cures sont évaluables à l'égard des critères adoptés, c'est-à-dire dans la mesure où l'activité réverse transcriptase a été mesurable pour le malade considéré et où sa valeur au jour 4 de la cure est encadrée par des valeurs connues au jour 15 de la cure précédente et au jour 15 de la cure considérée ;
- sur ces 8 cures évaluables, dans 7 cas la valeur au jour 4 est inférieure aux valeurs qui l'encadrent indiquant l'existence d'un inhibition nette de la réverse transcriptase ;
- sur ces 7 cas, dans 4 d'entre eux l'activité réverse transcriptase est ramenée à 0 (ou à une valeur négligeable, non significativement différente de 0) ;
- sur ces 7 cas, si l'on normalise les valeurs obtenues en les exprimant en % de la valeur la plus élevée mesurée pour le malade considéré, les moyennes obtenues sont les suivantes :
- RT au J15 de la cure précédente : 72,3
- RT au J4 de la cure précédente : 9,7
- RT au J15 de la cure considérée : 62,4

l'inhibition est donc très évidente au 4e jour de la cure ;

- ces 7 cas sont obtenus aux doses suivantes (en mg/m2/administration) 2 fois à 50, 3 fois à 200, 1 fois à 250 et 1 fois à 300.

Toutes ces doses sont cliniquement parfaitement tolérées.

L'ensemble de ces résultats démontre qu'en situation clinique l'administration de BD 40 trichlorhydrate est effectivement capable de provoquer une inhibition marquée de la réverse transcriptase, une enzyme indispensable à la réplication du virus, et que le BD 40 peut donc être un médicament approprié au traitement des états pathologiques dus à l'infection par le virus HIV. Des travaux ultérieurs préciseront les modalités optimales de traitement permettant d'obtenir le meilleur bénéfice thérapeutique.

Le BD 40 se présente donc comme un excellent agent antiviral dont l'action sur les virus HIV ainsi que la toxicité relativement faible observée chez l'homme, justifient son utilisation pour le traitement du SIDA.

Les composés de formule I définis ci-après ont également donné des résultats sensiblement analogues :

<u>BR 1376</u> $R_1$ : H

$R_2$ : $CH_3$

<u>BD 39</u> $R_1$ : Cl

$R_2$ : H

<u>BD 53</u> $R_1$ : 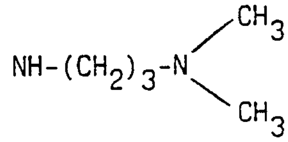

$R_2$ : H

BD 55 $R_1$ : 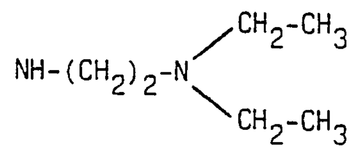

$R_2$ : H

BD 96 $R_1$ : 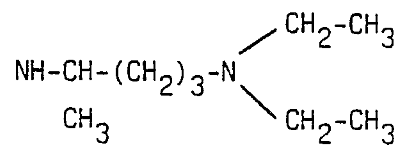

$R_2$ : H

BD 92 $R_1$ : $NH-(CH_2)_3-NH_2$

$R_2$ : H

## Revendications

1. Utilisation des dipyrido [4,3-b] [3,4-f] indoles de formule :

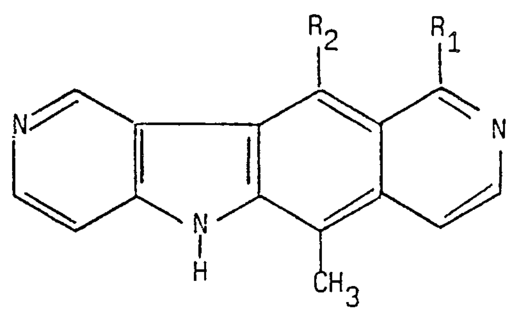

I

dans laquelle :

- $R_1$ est l'hydrogène, le groupe hydroxy, un groupe alkyle en $C_1$-$C_6$, un groupe alkyl($C_1$-$C_6$)thio, un groupe alcoxy en $C_1$-$C_6$, un halogène, de préférence le chlore ou un groupe amino non substitué ou substitué par un groupe de formule :

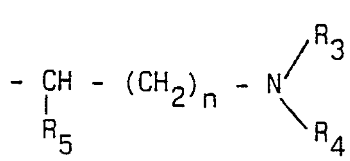

dans laquelle $R_3$ et $R_4$ identiques ou différents, représentent chacun l'hydrogène ou un radical alkyle en $C_1$-$C_6$, $R_5$ représente l'hydrogène ou un radical alkyle en $C_1$-$C_6$ et <u>n</u> représente un nombre entier de 1 à 10 ;

- $R_2$ est l'hydrogène ou un groupe alkyle en $C_1$-$C_4$, et de leurs sels pharmaceutiquement acceptables, pour la préparation de médicaments utiles pour le traitement du SIDA.

2. Utilisation selon la revendication 1, caractérisée en ce que les dipyrido [4,3-b] [3,4-f] indoles sont les composés répondant à la formule I dans laquelle :
- $R_1$ est l'hydrogène, le chlore ou un groupe de formule

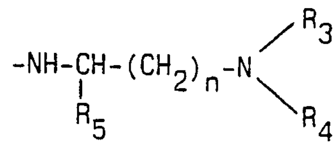

dans laquelle n est un nombre entier compris entre 1 et 4, $R_3$ et $R_4$ identiques ou différents, représentent l'hydrogène ou un groupe alkyle en $C_1$-$C_6$, de préférence en $C_1$-$C_4$ ; $R_5$ est l'hydrogène ou un groupe alkyle en $C_1$-$C_4$, de préférence le groupe méthyle,
- $R_2$ est l'hydrogène ou un groupe alkyle en $C_1$-$C_4$, de préférence le groupe méthyle ainsi que leurs sels pharmaceutiquement acceptables.

3. Utilisation selon l'une des revendications 1 ou 2, caractérisée en ce que le dipyrido [4,3-b] [3,4-f] indole est le 1- ( -diéthylaminopropyl)amino -5-méthyl-dipyrido [4,3-b] [3,4-f] indole (BD 40).

4. Utilisation selon la revendication 3, caractérisée en ce que le BD 40 est sous forme de trichlorhydrate.

FIG.1

INHIBITION DE LA REVERSE TRANSCRIPTASE DE $HIV_1$
PAR L'AGENT BD40

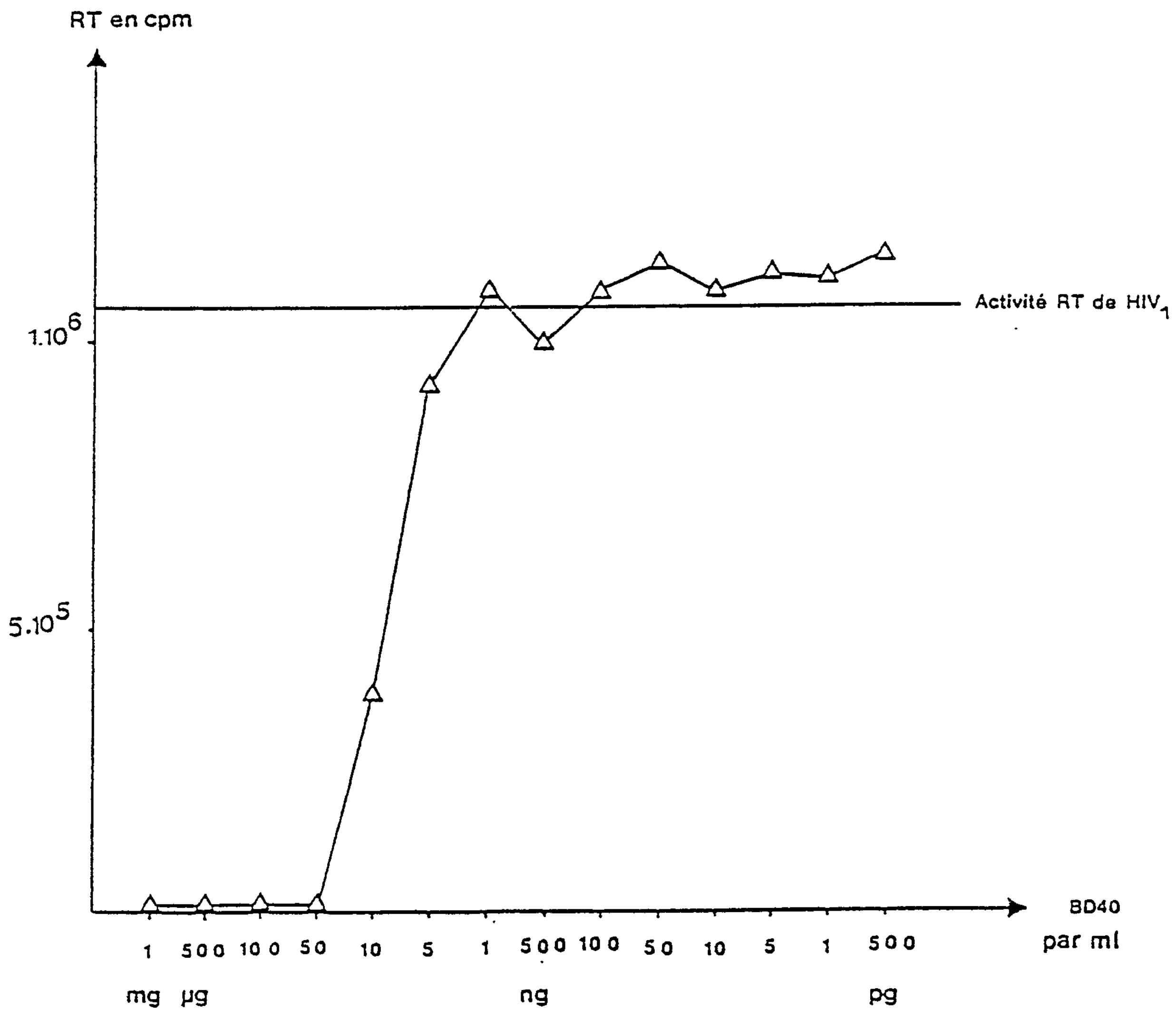

# FIG. 2

MECANISME D'ACTION DU BD40 SUR L'INFECTION
DE LYMPHOCYTES DU SANG PERIPHERIQUE PAR HIV

FIG. 2a

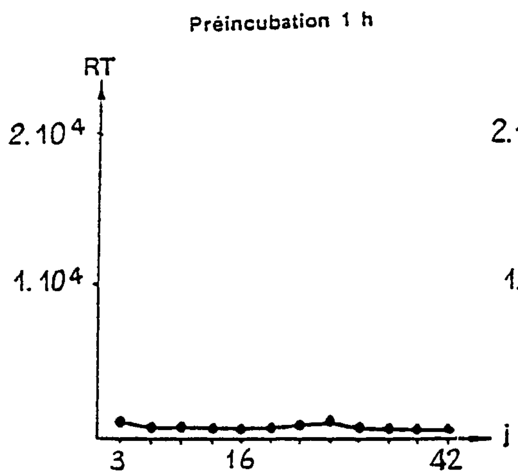


FIG. 2b

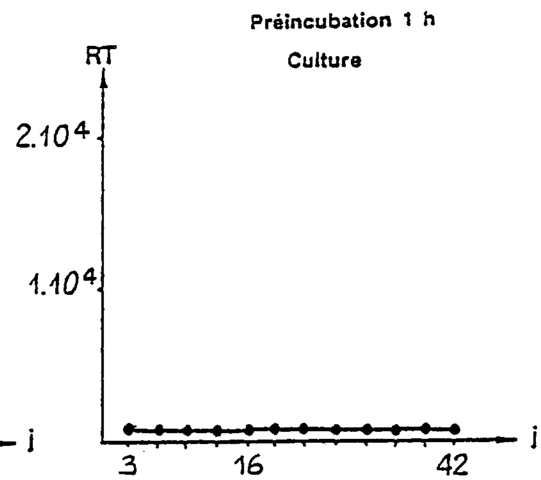


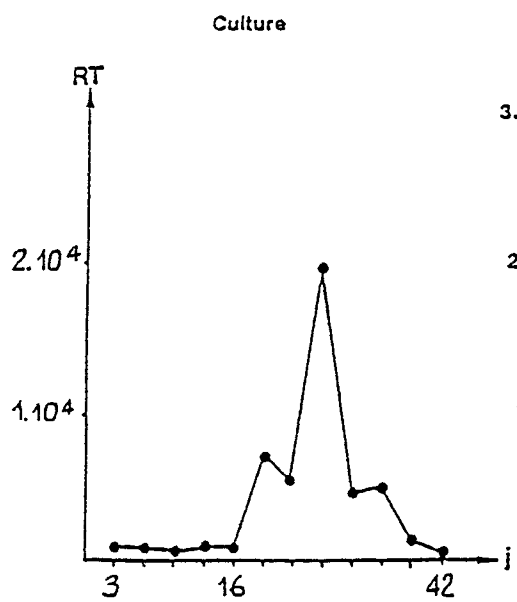


FIG. 2c

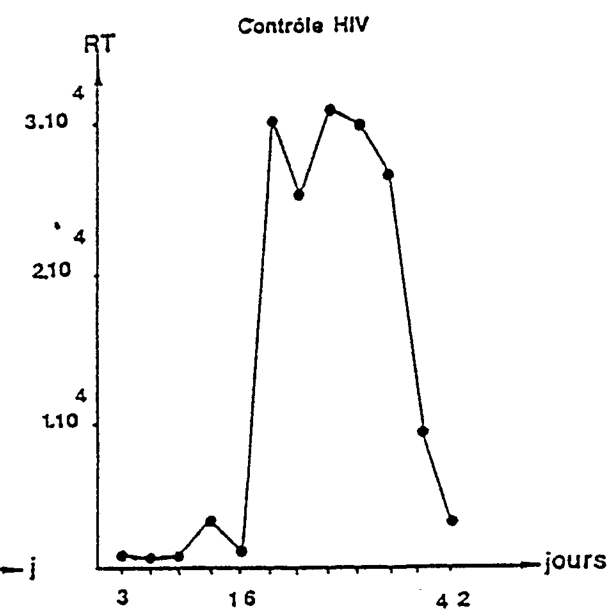


FIG. 2d

FIG. 3

ACTION DU BD40 SUR LA PRODUCTION DU VIRUS HIV
PAR LES LYMPHOCYTES PERIPHERIQUES